# EUROPEAN PATENT SPECIFICATION

(11) **EP 2 862 585 B1**
(45) Date of publication and mention of the grant of the patent: **28.02.2018**
(21) Application number: 13804358.3
(22) Date of filing: 13.06.2013
(51) Int. Cl.: A61M 5/158, A61M 25/06, A61M 5/32, A61M 1/36, A61B 17/34

(54) **PUNCTURE NEEDLE**
PUNKTIONSNADEL
AIGUILLE DE PONCTION

(30) Priority: 14.06.2012 JP 2012134902
(43) Date of publication of application: 22.04.2015
(73) Proprietor: Asahi Kasei Medical Co., Ltd., Chiyoda-ku Tokyo 101-8101 (JP); Nextier Corporation, Toyohashi-shi, Aichi 441-8107 (JP)
(72) Inventor: SHINZATO, Toru, Nagoya-shi Aichi 465-0048 (JP); MIWA, Masamiki, Kitanagoya-shi Aichi 481-0042 (JP); MARUYAMA, Yasuyo, Toyohashi-shi Aichi 441-8107 (JP); SASAKI, Masatomi, Tokyo 101-8101 (JP)
(74) Representative: dompatent von Kreisler Selting Werner - Partnerschaft von Patent- und Rechtsanwälten mbB
(86) International application number: PCT/JP2013/066380
(87) International publication number: WO 2013/187483

(56) References cited:
- EP-A1- 1 297 856
- JP-A- 2011 250 999
- US-A- 4 808 170
- US-A1- 2011 295 152

## Description

### Technical Field

The present invention relates to a puncture needle that is inserted, via a puncture route formed to cover from a skin surface to a shunt vessel surface, into an arc-shaped or linear puncture hole already made on a shunt vessel wall, thereby, while beginning at both ends of an incision that forms the puncture hole, dissecting the shunt vessel wall so as to expand the incision of the puncture hole.

### Background Art

When performing blood purification such as hemodialysis, two puncture needles, each having a sharply pointed end, are used to puncture a shunt, at different parts thereof, from a skin surface through hypodermis; blood is removed from the shunt vessel through one of such puncture needles; such removed blood is purified in a blood purifier; and then, such purified blood is returned to the shunt vessel through the other puncture needle.

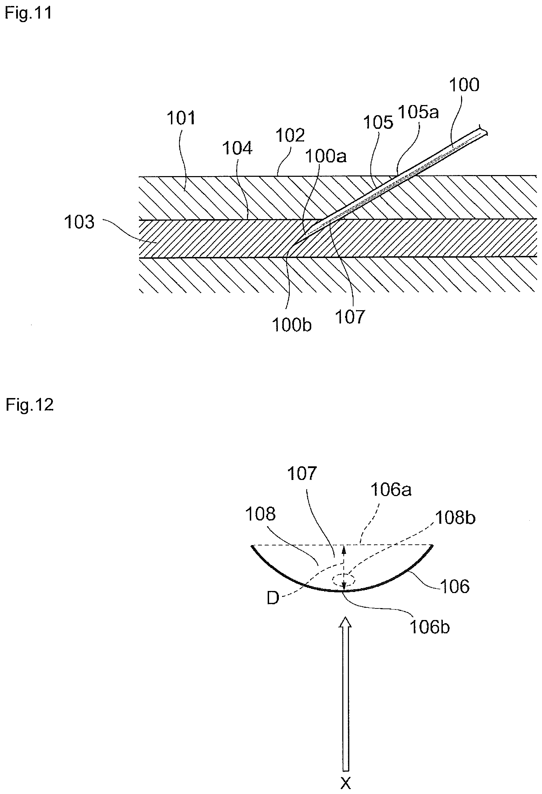

The above puncture operation needs to be performed every time blood purification is conducted, and this causes a patient significant pain on each such occasion. In order to reduce the pain produced when making a puncture, therefore, a so-called buttonhole puncture method has been used in recent years (see Patent Document 1 and Non-patent Document 2). In the buttonhole puncture method, as shown in, for example, Fig. 11, during the first puncture, an ordinary puncture needle 100 having high sharpness is used to obliquely penetrate hypodermis 101 through skin 102 and then puncture a shunt vessel 103 at an angle of approximately 30° while an inclined end face of the ordinary puncture needle 100 is facing upward. Thus, a puncture route 105, which covers from the skin 102 to a surface 104 of the shunt vessel 103, is made, and an arc-shaped incision 106, which is convex backward in a puncture direction X, and a puncture hole 107, as are shown in, for example, Fig. 12, are formed in the surface 104 of the shunt vessel 103 and also on an extension of the puncture route 105. It should be noted that, in this specification, "back" refers to the back in the puncture direction X, and "front" refers to the front in the puncture direction X.

In the incision 106 formed as described above, a flap refers to an area, on a wall of the shunt vessel, which is defined by a virtual line 106a connecting both ends of the arc-shaped incision 106 and the arc-shaped incision 106. A flap 108 functions as a cover of the puncture hole 107, and a part of the virtual line 106a functions as a hinge for the opening and closing of the flap 108.

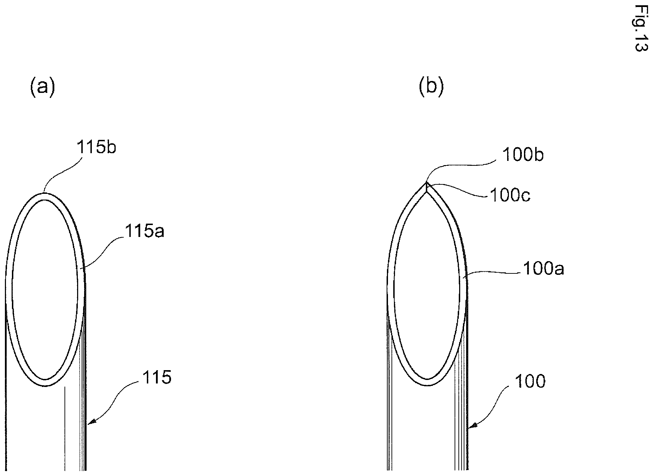

After the puncture route 105 and the puncture hole 107 are made via the first puncture by the ordinary puncture needle 100 having high sharpness, every time blood purification is performed, as shown in Fig. 13(a), a puncture needle (hereinafter referred to as a "dull needle") 115 having low sharpness at both a front end edge and side edges of an inclined end face thereof is inserted, through a skin-side entrance 105a shown in Fig. 11, into the puncture route 105, and is then caused to pass, via the puncture route 105, through the puncture hole 107 which is formed by the incision 106 and which is located on the surface 104 of the shunt vessel 103, and is then inserted into the shunt vessel 103. According to this method, the skin 102 does not need to be punctured anew after the puncture route 105 is made, and therefore, a patient's pain will be reduced.

It should be noted that, as shown in Fig. 13(b), a pointed cutting line 100c is generally formed at a front end of the ordinary puncture needle 100, and accordingly, the front end of the puncture needle 100 serves as a pointed end point 100b, whereby the pointed end point 100b is capable of penetrating skin, hypodermis and vessel walls. Meanwhile, as shown in Fig. 13(b), the dull needle 115 has an inclined end face 115a, which is formed by obliquely cutting a tubular front end part. A front end part 115b of the inclined end face 115a is a part of the inclined end face 115a which has an elliptical shape and roundness. Therefore, the front end part 115b neither sticks into skin nor penetrates skin, hypodermis and vessel walls.

In the meantime, in the case in which, after the first puncture with the ordinary puncture needle 100 having high sharpness, the dull needle 115 is inserted into the incision 106 formed as described above, firstly, as described above, the front end part 115b of the dull needle 115 is inserted into the puncture route 105 through the skin-side entrance 105a, is then moved forward inside the puncture route 105, and is then caused to reach the surface of the shunt vessel 103. When the front end part 115b of the dull needle 115 has reached the surface of the shunt vessel 103, the front end part 115b of the dull needle 115 senses a bottom point 106b of the incision 106 shown in Fig. 12, which has been formed in the surface 104 of the shunt vessel 103, as an uneven part due to a fibrin adhesive that welds the bottom point 106b. Then, the front end part 115b of the dull needle 115 is slid toward a part immediately in front of the bottom point 106b, an optimum pressure-application part 108b on the flap 108. When the front end part 115b of the dull needle 115 reaches the optimum pressure-application part 108b, the dull needle 115 is erected in a vertical direction, and pressure is applied to the optimum pressure-application part 108b on the flap 108 using the front end part 115b of the dull needle 115. Thus, the flap 108 is rotated inside a lumen of the shunt vessel 103 so as to open the puncture hole 107, and at the same time, the front end part 115b of the dull needle 115 is caused to pass into the shunt vessel 103.

However, it is often impossible for the front end part 115b of the dull needle 115 to sense the bottom point 106b of the incision 106, and thus, the front end part 115b of the dull needle 115 may pass the flap 108, or when erecting the dull needle 115 in a vertical direction at the optimum pressure-application part 108b, the front end part 115b of the dull needle 115 may slide out from the optimum pressure-application part 108b to another part. In such case, the flap 108 is not rotated inside the lumen of the shunt vessel 103, and the dull needle 115 is thus not inserted into the shunt vessel 103.

### Prior Art References

### Patent Document

Patent Document 1: Japanese Patent Laid-Open No. 2009-045124

### Non-Patent Document

Non-patent Document 1: Shigeki Toma, Takahiro Shinzato, Kenji Maeda et al., "A timesaving method to create a fixed puncture route for the buttonhole technique," Nephrol Dial Transplant, UK, Oxford University Press, 2003, 18: p2118-2121 EP1297856 and US2011/0295152 also describe prior art puncture needles.

### Summary of the Invention

### Problem to be Solved by the Invention

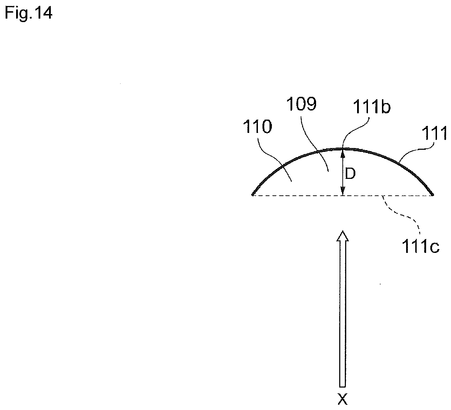

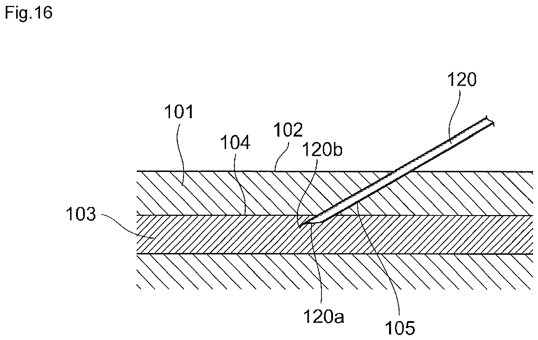

In order to solve this issue, an arc-shaped incision 111, which is convex forward in a puncture direction X, as shown in Fig. 14, is formed in the surface 104 of the shunt vessel 103 and also on the extension of the puncture route 105. In order to form the convex forward arc-shaped incision 111, for the first puncture, for example, a puncture needle 120 (hereinafter referred to as a "curved-and-pointed end needle") is used which has an inclined end face 120a and is provided, at a frond end of the inclined end face 120a, with a pointed end point 120b and in which the pointed end point 120b is curved with respect to the direction of a centerline Y. When the curved-and-pointed end needle 120 is used to puncture the shunt vessel 103 through the skin 102, firstly, as shown in Fig. 16, the curved-and-pointed end needle 120 is obliquely inserted into the hypodermis 101 through the skin 102 at an angle of approximately 30° while the inclined end face 120a of the curved-and-pointed end needle 120 is facing downward. Then, the inclined end face 120a of the curved-and-pointed end needle 120 is pushed toward the shunt vessel 103. Further, even after the pointed end point 120b has reached the surface 104 of the shunt vessel 103, the inclined end face 120a of the curved-and-pointed end needle 120 is moved forward into the shunt vessel 103 while maintaining the posture, whereby the pointed end point 120b penetrates a shunt vessel wall.

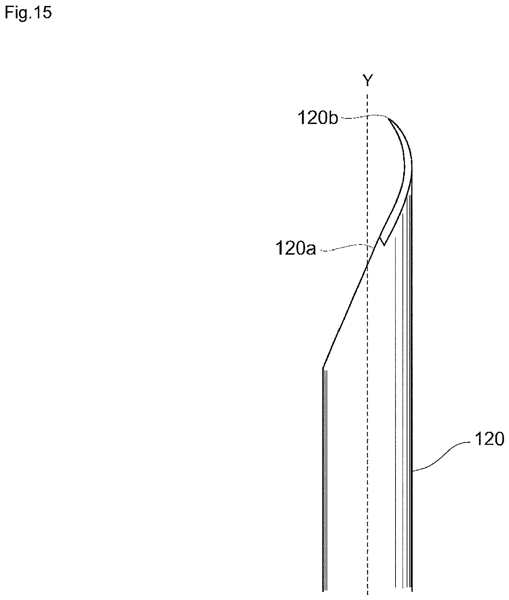

Regarding an arc-shaped puncture 109 which is convex forward in the puncture direction X and a flap 110 covering the puncture hole 109, which are shown in Fig. 14 and which are made by using the curved-and-pointed end needle 120 shown in Fig. 15, since a front end of the flap 110 is located forward in the puncture direction X, when inserting the front end part 115b of the dull needle 115 into the shunt vessel through the puncture hole 109, as long as the front end part 115b is slid along the shunt vessel wall 104 while applying pressure to the flap 110 on the shunt vessel wall 104, the front end part 115b of the dull needle 115 naturally enters the shunt vessel 103 through the puncture hole 109 on the shunt vessel wall 104 in many cases. This brings about the advantage that there is no need to employ a technique of erecting, on the flap 110, the dull needle 115 in a vertical direction and then applying pressure to the flap.

However, even in the case of making the puncture hole 109 with the use of the curved-and-pointed end needle 120, the insertion of the dull needle 115 into the puncture hole 109 may still not be performed properly. Thus, the inventors of the present invention thought that the ease of insertion of the dull needle 115 into the puncture hole 109 would be improved by expanding the area of the puncture hole 109. The area of the puncture hole 109 is correlated with a distance D between a bottom point 111b of the convex forward arch-shaped incision 111 forming the puncture hole 109 and a virtual line 111c connecting both ends of the arc.

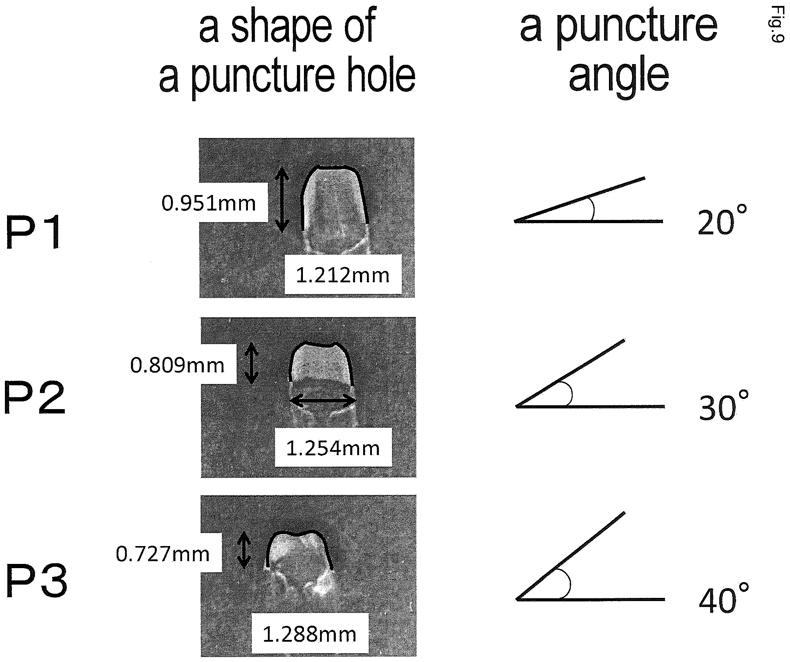

In order to solve the above-described issue, the inventors of the present invention have conducted intensive studies into the factors that determine the distance D between the bottom point 111b of the convex forward arc-shaped incision 111 made by the curved-and-pointed end needle 120 and the virtual line 111c connecting both ends of the arc. As a result, it has been found that, as shown in Fig. 9, in the case of making a puncture with the curved-and-pointed end needle 120, the smaller the angle formed by a puncture direction and a shunt vessel wall becomes, the longer the distance D between the bottom point 111b of the incision 111 forming the puncture hole 109 and the virtual line 111c connecting both ends of the arc becomes.

The above study results show that, in order to make, on a shunt vessel wall, a greater-area puncture hole which makes it easier to perform an insertion of a dull needle, it is sufficient to puncture, with the curved-and-pointed end needle 120, a shunt vessel so as to attain a smaller angle formed by the curved-and-pointed end needle 120 with a skin surface, i.e., in the state in which the curved-and-pointed end needle 120 is more inclined toward a skin surface. However, if the curved-and-pointed end needle 120 punctures a shunt vessel at an angle of less than 30°, the tubular part of the curved-and-pointed end needle 120 obstructs the view of the operator who sees a punctured part of a skin surface, and thus, it becomes technically difficult for the pointed end 120b of the curved-and-pointed end needle 120 to puncture the skin surface.

Therefore, it is difficult for the curved-and-pointed end needle 120 to have an angle smaller than an ordinary angle of approximately 30°. Namely, with the above method, it is difficult to increase the distance D between the bottom point 111b of the incision 111 forming the puncture hole 109 and the virtual line 111c connecting both ends of the incision 111, thereby enlarging the area of the puncture hole 109.

The present invention has been made in view of the above circumstances, and an object of the present invention is to provide a puncture needle that is inserted into an incision and a puncture hole, which are formed by making an insertion of a needle for forming a puncture route at an ordinary angle of approximately 30° with respect to a skin surface, thereby enabling the expansion of such incision and such puncture hole.

### Means for Solving the Problem

A gist of the present invention resides in a puncture needle that is inserted into a puncture route formed to cover from a skin surface to a shunt vessel surface so as to expand an incision forming a puncture hole that is made in the shunt vessel surface, the puncture needle comprising: an inclined end face that is inclined toward a front end of the needle, wherein a front end part of the inclined end face constitutes an edge having no sharpness, and wherein right and left side edges of the inclined end face which are contiguous with the front end part are provided with cutting lines each having an edge angle from 5 degrees to 85 degrees. It should be noted that the "edge having no sharpness" refers to an edge obtained by subjecting a sharp cutting edge to a rounding process, such as chamfering or providing such cutting edge with a curvature whose center of curvature is inside the cutting edge.

When using the puncture needle of the present invention, a puncture route is made in advance so as to cover from the skin to a shunt vessel surface, and a puncture hole that is convex forward in a puncture direction is also made in advance in the shunt vessel surface. The puncture needle of the present invention is then inserted into the above puncture route, and moved forward along the puncture route and then inserted into the puncture route formed by the above incision while maintaining the posture. With such operation, cutting lines forming side edges of an inclined end face of the puncture needle of the present invention dissect a shunt vessel wall obliquely backward in the puncture direction or backward therein while beginning at both ends of the incision forming the already-made puncture hole. Thus, the distance between a bottom point of the incision and a virtual line connecting both ends of the incision is extended, resulting in an expansion of the area of the puncture hole. Further, at this point, a front end part of the puncture needle of the present invention has an edge having no sharpness, and thus, the front end part of the puncture needle of the present invention will not stick on a wall of the already-made puncture route. Therefore, the front end part of the puncture needle of the present invention smoothly moves forward to the puncture hole made on the shunt vessel wall along the already-made puncture route. Further, as the front end part of the puncture needle of the present invention constitutes an edge having no sharpness, the puncture needle of the present invention that has entered the shunt vessel through the puncture hole of the shunt vessel surface will not impair a backside wall of the shunt vessel.

A gist of the present invention resides in a puncture needle wherein right and left side parts of the inclined end face which are contiguous with the front end part are respectively inclined, and side edges of the side parts are provided with the cutting lines. The side parts may be inclined so as to become lower in an outward direction. Further, a gist of the present invention resides in a puncture needle wherein the front end part of the inclined end face is formed into an arc shape.

A gist of the present invention resides in a puncture needle wherein the front end part of the inclined end face has an arc-direction length of 0.05 mm or more and less than 1.2 mm. As the front end part has a length of 0.05 mm or more, even when such front end part hits a part other than the puncture hole on the shunt vessel wall, such front end part is prevented from impairing such hit part. Further, as the front end part has a length of less than 1.2 mm, it becomes easy for such front end part to be inserted into the entrance of the puncture route on the skin surface.

A gist of the present invention resides in a puncture needle wherein the arc-shaped front end part of the puncture needle has a radius of curvature of 200 µm or more and less than 4.4 mm. As such radius of curvature is 200 µm or more, the front end part of the puncture needle can be prevented from damaging the shunt vessel wall. Further, as the radius of curvature of the front end part of the puncture needle is less than 4.4 mm, it is easy for the front end to be inserted into the entrance of the puncture route on the skin surface.

A gist of the present invention resides in a puncture needle wherein start points of the cutting lines are located at boundaries between the front end part of the inclined end face and the side parts thereof, and end points of the cutting lines are located at positions where a width-direction virtual transverse line passing through a point that falls, with respect to a front end of the inclined end face, within the range of one-tenth to seven-tenths of a distance that covers from the front end to a back end of the inclined end face intersects with the side edges of the inclined end face. As the inclined end face has, at parts where the cutting lines are formed, a larger traverse diameter with respect to the diameter of the tubular part, the incision is more extended, and it is therefore preferable for the inclined end face to have, at parts where the cutting lines are formed, a larger traverse diameter with respect to the diameter of the tubular part. However, if the end points of the parts where the cutting lines are formed are located at positions where a virtual transverse line passing through a point of less than one-tenth of the distance that covers from the front end and back end of the inclined end face intersects with the side edges of the inclined end face, because the inclined end face has a small transverse diameter at the end points of the parts where the cutting lines are formed, when inserting the puncture needle of the present invention into an already-formed incision, such incision cannot be extended sufficiently. Meanwhile, if the end points of the parts where the cutting lines are formed are located at positions where a virtual transverse line passing through a point of equal to or more than seven-tenths of the distance that covers from the front end and back end of the inclined end face intersects with the side edges of the inclined end face, in the case where the puncture of the present invention is placed at rest while an inclined end face thereof faces upward and is seen from a side surface thereof, a side edge part of the inclined end face provided with a cutting line has a downward convex arc shape in terms of the manufacturing technique, and a part near an end point of such part will get caught on an end of an incision.

A gist of the present invention resides in the edge angle of the cutting lines being from 5 degrees to 85 degrees, preferably from 15 degrees to 65 degrees. When the edge angle of the cutting lines is less than 5 degrees, the strength of the cutting lines becomes too weak. Meanwhile, when the edge angle of the cutting lines is 85 degrees or more, the sharpness becomes too low.

Further, the puncture needle may be inserted into / puncture the already-made puncture hole. Moreover, the puncture needle may have a diameter greater than that of a puncture needle for forming the puncture hole. Furthermore, regarding the puncture needle, an inclined surface of each of the side parts may be a flat surface. A needle outer-diameter width of a part of an inclined surface of each of the side parts which is inclined outward may be smaller than a needle outer-diameter width of a part behind the part of the inclined surface.

### Effect of the Invention

According to the present invention, a dull needle is inserted into an already-made puncture hole, thereby expanding such puncture hole, and accordingly, the success rate of the insertion of a dull needle into a puncture hole can be improved.

### Brief Description of the Drawings

Fig. 1 is a diagram illustrating the outline of a structure of a blood purification treatment device.
Fig. 2A is a top view illustrating a part around and encompassing a front end of a puncture needle of the present invention.
Fig. 2B is a top view illustrating a part around and encompassing a front end of a dull needle.
Fig. 2C is a top view illustrating another example of a part around and encompassing a front end of a puncture needle.
Fig. 3 is a perspective view illustrating the part around and encompassing the front end of the puncture needle of the present invention.
Fig. 4A is a side view of the part around and encompassing the front end of the puncture needle of the present invention.
Fig. 4B is an enlarged side view of a part around and encompassing a front end having no sharpness of the puncture needle of the present invention.
Fig. 5 is an explanatory diagram illustrating the state in which the puncture needle of the present invention is being inserted into a puncture route.
Fig. 6 is a view illustrating the shape of an incision in which both ends thereof are extended.
Fig. 7 is an explanatory diagram illustrating the state in which the dull needle is being inserted into a puncture route.
Fig. 8 is a diagram illustrating the state in which the dull needle is being inserted into a puncture hole on a shunt vessel wall.
Fig. 9 is a photograph of a puncture hole made on a silicone rubber plate which shows the relationship between a puncture angle of a puncture needle and a shape of a puncture hole formed by the puncture needle.
Fig. 10 is a photograph illustrating an incision made in a silicone rubber plate.
Fig. 11 is an explanatory diagram illustrating the state in which a puncture route is formed by an ordinary puncture needle.
Fig. 12 is a diagram illustrating the shape of a convex backward incision.
Fig. 13(a) is a top view of a front end part of the dull needle, and Fig. 13(b) is a top view of a front end part of the ordinary puncture needle.
Fig. 14 is a diagram illustrating the shape of a convex forward incision.
Fig. 15 is a side view of a curved-and-pointed end needle.
Fig. 16 is an explanatory diagram illustrating the state in which a puncture route and a puncture hole are formed by the curved-and-pointed end needle.

### Mode for Carrying out the Invention

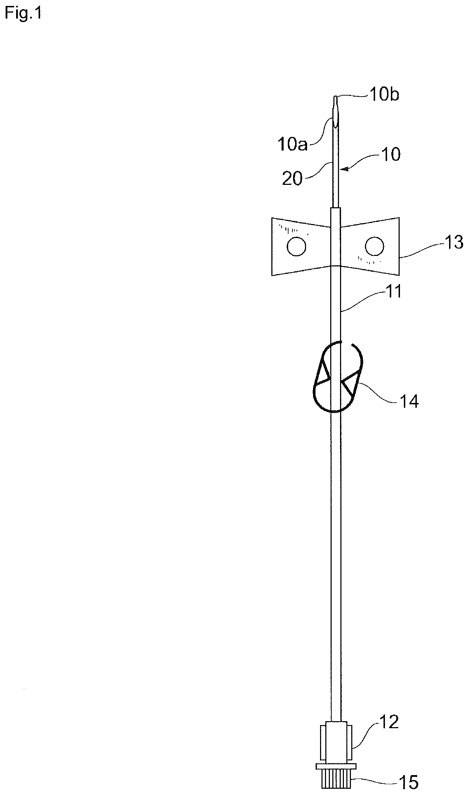

A preferred embodiment of the present invention will hereinafter be described with reference to the drawings. Fig. 1 is an explanatory diagram illustrating an example of a blood purification treatment device 1 having a puncture needle 10 of the present invention.

The blood purification treatment device 1 comprises: the puncture needle 10, which makes a puncture in a patient during a second puncture following a first puncture with, for example, a curved-and-pointed end needle; a tube 11 connected to a back end part of the puncture needle 10; and a connecting part 12 for connecting a back end part of the tube 11 to another tube. A holding part 13 that is held by an operator when moving the puncture needle 10 is provided at a part close to the puncture needle 10 of the tube 11. Further, the tube 11 is mounted with a clamp 14. A cap 15 is fitted onto the connecting part 12.

The blood purification treatment device 1 is connected to another tube by means of, for example, the connecting part 12, so as to configure a part of a blood purification circuit having a blood purifier (not shown). The blood purification treatment device 1 is attached to each of a blood-removal side end section and a blood-return side end section of the blood purification circuit, and such blood purification treatment device 1 removes or returns blood through the puncture needle 10 at a front end of the blood purification treatment device 1 during blood purification treatment. It should be noted that examples of blood purification treatment include dialysis treatment, plasma exchange treatment, plasma adsorption treatment and blood component elimination treatment.

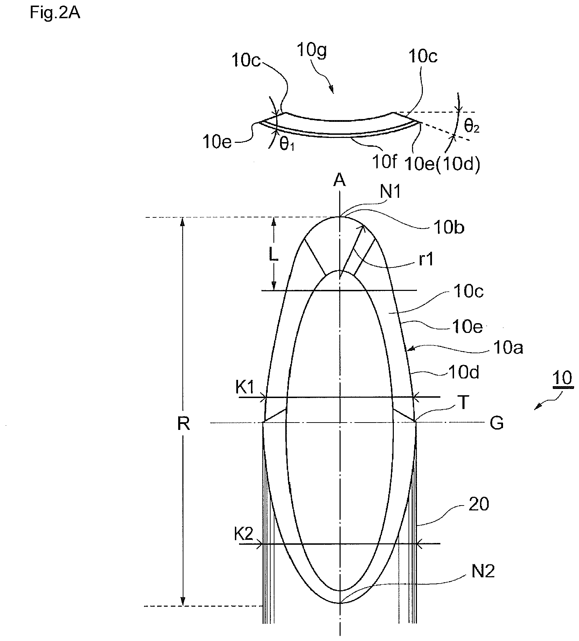

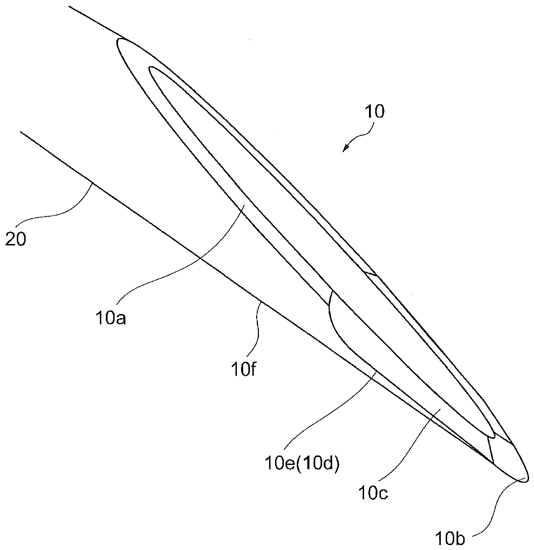

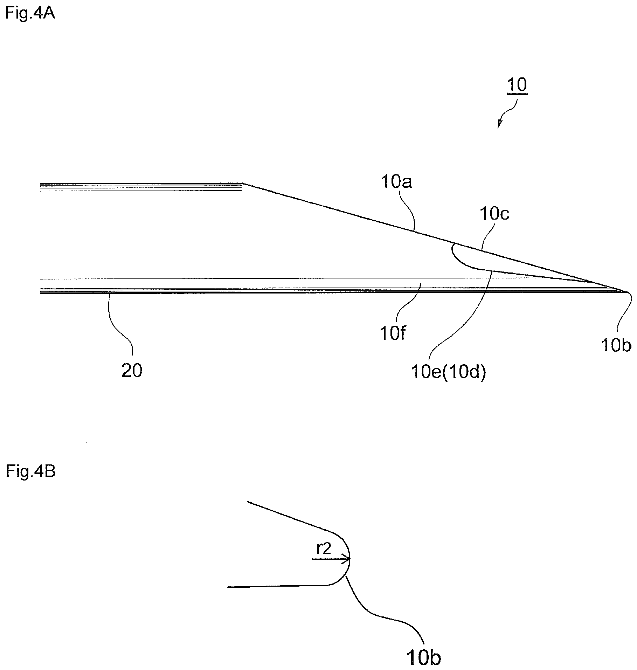

As shown in Figs. 2A, 3 and 4A, the puncture needle 10 has a tubular part 20 having an inclined end face 10a that is inclined toward a front end of the needle, and a front end part 10b of the inclined end face 10a has a convex forward arch shape and an edge having no sharpness. More specifically, as to the front end part 10b, it is desirable for a radius of curvature r2 thereof, when seen from a side surface of the needle, to have no sharpness within the range of 0.01 to 0.05 mm, as shown in Fig. 4B. More preferably, it is desirable for the radius of curvature r2 to fall within the range of 0.015 to 0.03 mm. As one example, the radius of curvature r2 of the front end part 10 is set at 0.02 mm. Further, the length of an arc forming the front end part 10b is set at between 0.05 mm or more and less than 1.2 mm, e.g., 0.6 mm, and a radius of curvature of the arc of the front end part 10b when seen from above the needle is set at between 200 µm or more and less than 4.4 mm, e.g., 0.43 mm.

Further, right and left side parts 10c contiguous with the arc-shaped front end part 10b of the puncture needle 10 are each inclined such that an outer part thereof is low, and side edges 10d of the side parts 10c are provided with sharp cutting lines 10e. Further, as shown in Fig. 2A, an edge angle (θ1) of the cutting lines 10e is formed at an angle from 5 degrees to 85 degrees and has a cutting property. End points T behind parts that form the sharp cutting lines 10e are located at the intersection of a virtual transverse line G, which passes through a point that falls within the range of one-tenth to seven-tenths of a distance R, which covers from a front end N1 to a back end N2 of the inclined end face 10a, and the side edge 10d of the inclined end face 10a. Further, an inclined surface (upper surface) that is inclined toward the outer part of the side part 10c is not curved but serves as a flat surface. An outer diameter width K1, of the puncture needle 10, at a certain part of such inclined surface that is inclined toward the outer part of the side part 10c is narrower than an outer diameter width K2, of the puncture needle 10, at a part behind such certain part of the inclined surface. Further, an inclination angle θ2 of the inclined surface that is inclined toward the outer part of the side part 10c is preferably more than 0 degree and equal to or less than 30 degrees.

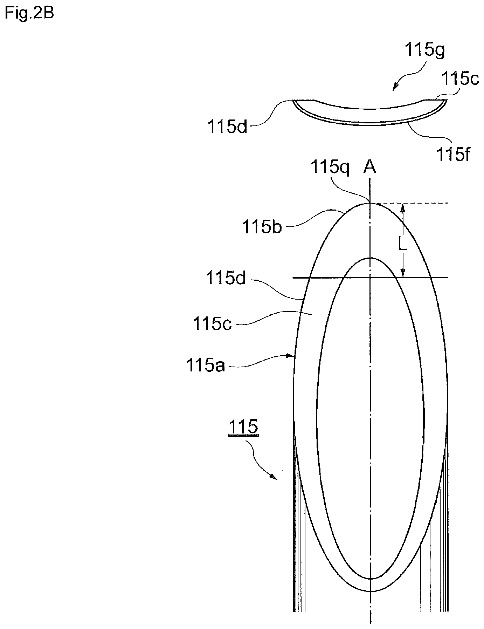

Here, in order to clarify the feature of the puncture needle 10 based on the comparison with the dull needle 115, Fig. 2B shows, in the lower part thereof, the shape of the inclined end face 115a of the dull needle 115 which is placed at rest while the inclined end face 115a faces upward and which is seen from above, and Fig. 2B also shows, in the upper part thereof, a cross-section 115g of the inclined end face 115a orthogonal to a long axis A of the dull needle, the cross-section 115g being apart, by any length L, from a front end point 115q of the inclined end face 115a. Meanwhile, Fig. 2A shows, in the lower part thereof, the shape of the inclined end face 10a of the puncture needle 10 which is placed at rest while the inclined end face 10a faces upward and which is seen from above, and Fig. 2A also shows, in the upper part thereof, as in the case of the dull needle 115, a cross-section 10g of the inclined end face 10a orthogonal to a long axis A of the puncture needle 10, the cross-section 10g being located at a part which is apart, by any length L, from the front end N1 of the inclined end face 10a and in which the side edges 10d are provided with the cutting lines 10e.

In the case of the dull needle 115, as shown in the lower part of Fig. 2B, the shape of the inclined end face 115a is generally elliptical, and the front end part 115b of the inclined end face 115a is a part of such elliptical shape and thus has a convex forward arc shape in a puncture direction. Further, as shown in the upper part of Fig. 2B, in such state, right and left side parts 115c of the inclined end face 115a of the dull needle 115 are formed horizontally. Therefore, the angle of each of side edges 115d, which is formed by an outer surface 115f of the dull needle 115 and the inclined end face 115a thereof, is an obtuse angle, and thus, when the dull needle 115 is inserted into a shunt vessel, a wall of the shunt vessel will not be laterally dissected.

Meanwhile, the side part 10c of the inclined end face 10a of the puncture needle 10 shown in the upper part of Fig. 2A is obliquely polished or cut so as to be inclined in an outward direction. Therefore, the angles of the side edges 10d, which are formed by the side parts 10c of the inclined end face 10a, obliquely formed so as to be inclined in an outward direction, and an outer surface 10f of the puncture needle 10, constitute the sharp cutting lines 10e. That is, when the inclined end face 10a of the puncture needle 10 is inserted into the shunt vessel 103, the sharp cutting lines 10e dissect the shunt vessel wall 104 in a backward direction.

Next, an explanation will be provided regarding a puncture operation for expanding the area of a puncture hole in a shunt vessel wall with the use of the puncture needle 10 configured as above. The first puncture operation for forming a puncture route and a puncture hole is performed prior to the performance of such puncture operation. For example, as shown in Fig. 16, in the state in which an inclined end face 120a of a curved-and-pointed end needle 120 faces downward, the curved-and-pointed end needle 120 is used to penetrate the hypodermis 101 through the skin 102 and then puncture the shunt vessel 103 at an angle of approximately 30° with respect to the shunt vessel surface 104. As a result, the puncture route 105 is formed between the skin 102 and the shunt vessel surface 104, and the puncture hole 109 formed by the convex forward arc-shaped incision 111, shown in Fig. 14, is made in the shunt vessel wall 104 on the extension of the puncture route 105.

Such puncture of the shunt vessel 103 by the curved-and-pointed end needle 120 is performed at two locations on the shunt vessel surface 104. From among two curved-and-pointed end needles 120, blood is removed from the shunt vessel 103 through one curved-and-pointed end needle 120; such removed blood is then purified in a blood purifier; and such purified blood is then returned to the shunt vessel 103 through the other curved-and-pointed end needle 120.

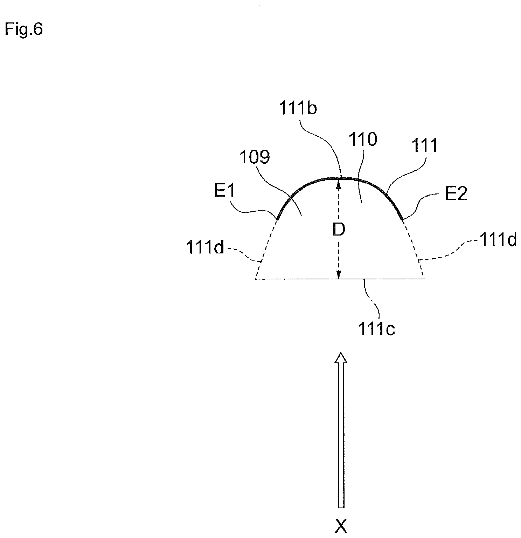

Subsequently, the second puncture operation with the puncture needle 10 is performed. In the second puncture operation, firstly, as shown in Fi g. 5, the puncture needle 10, which is thicker than the curved-and-pointed end needle 120, is inserted into the puncture route 105 through the entrance 105a of the puncture route 105 on the skin 102 while the inclined end face 10a is facing downward, and the puncture needle 10 is moved forward while maintaining the posture along the puncture route 105. Next, when the front end N1 of the puncture needle 10 has reached the flap 110 that covers the puncture hole 109 on the surface 104 of the shunt vessel 103, with such posture being maintained, the inclined end face 10a of the puncture needle 10 is inserted into the puncture hole 109. Then, the cutting lines 10e of the side parts 10c of the puncture needle 10 come into contact with both ends of the incision 111 of the puncture hole 109, and the cutting lines 10e dissect the shunt vessel surface 104 backward in the puncture direction X while beginning at both ends E1 and E2 of the incision 111 that forms the puncture hole 109 shown in Fig. 6, whereby the incision 111 is extended backward in the puncture direction X by an amount indicated by dotted lines 111d in Fig. 6. As a result, the arc depth of the incision 111, which serves as the distance D between the virtual line 111c connecting the ends of the incision 111 and the bottom point 111b of the incision 111, becomes greater, thereby resulting in the expansion of the area of the puncture hole 109. It should be noted that blood purification treatment is performed here by removing or returning blood through the puncture needle 10 placed inside the shunt vessel 103 as in the above-described manner.

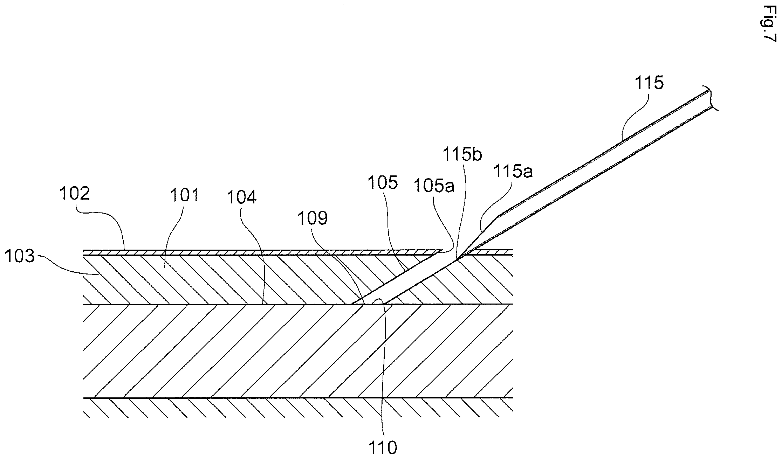

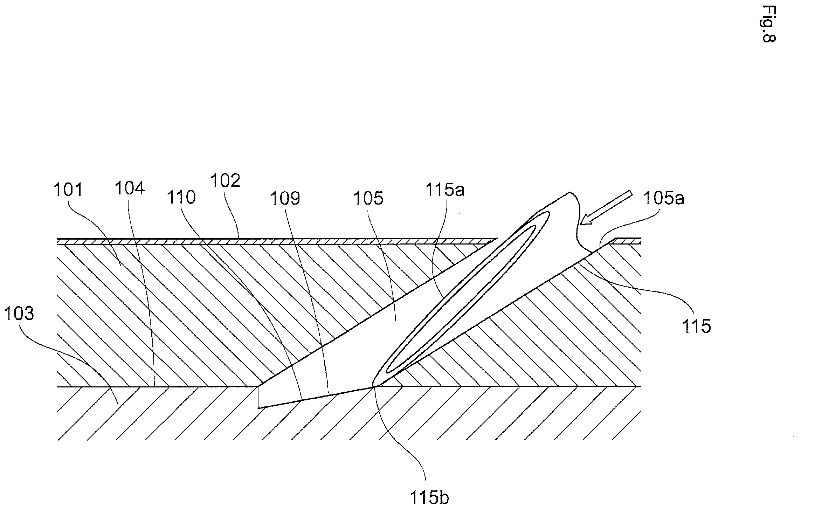

Next, the third and subsequent puncture operations are performed using the dull needle 115. In the third and subsequent puncture operations, the dull needle 115 having the same thickness as that of the puncture needle 10 used in the second puncture operation is inserted through the entrance 105a of the puncture route 105 on the skin 102 shown in Fig. 7 while the inclined end face 115a is facing upward. Subsequently, when the dull needle 115 is moved forward along the puncture route 105, the front end part 115b of the inclined end face 115a of the dull needle 115 reaches the surface 104 of the shunt vessel 103. Then, as shown in Fig. 8, the front end part 115b of the dull needle 115 presses the flap 110 downward into the shunt vessel 103, whereby the puncture hole 109 is opened, and the dull needle 115 is then inserted into the shunt vessel 103.

According to this embodiment, the incision 111 forming the puncture hole 109 extends downward in the puncture direction X, whereby the distance D between the virtual line 111c connecting the ends of the incision 111 and the bottom point 111b of the incision 111 is extended, leading to an expansion of the area of the puncture hole 109. It therefore becomes easy for the front end of the dull needle 115 to enter the puncture hole 109, resulting in an improvement in the success rate of the insertion of the dull needle 115 into the puncture hole 109. Further, although the performance of the initial puncture with the use of the thick curved-and-pointed end needle 120 causes strong puncture pain, if the narrower curved-and-pointed end needle 120 is used in the initial puncture and the thicker puncture needle 10 is then used to enlarge the puncture route 105 during the second puncture operation, the puncture pain caused in the first puncture operation becomes weaker, and hardly any puncture pain is caused during the second puncture operation. That is, with the use of the narrower curved-and-pointed end needle 120 for the first puncture and the thicker puncture needle 10 for the second puncture, the suffering felt by a patient due to puncture pain can be kept to a minimum.

In the above embodiment, the curved-and-pointed end needle 120 used for the first puncture and the puncture needle 10 used for the second puncture may have the same thickness. During the second puncture, when the puncture needle 10 is inserted into the puncture hole 109 made by the curved-and-pointed end needle 20 in the first puncture, if such insertion is performed at an angle in which the puncture needle 10 is more steeply inclined, i.e., at a smaller angle of the puncture needle 10 with respect to the skin surface, then, as shown in Fig. 6, the incision forming the puncture hole 109 is dissected obliquely backward in the puncture direction or backward therein while beginning at both ends of the incision, leading to the extension of the distance between the virtual line 111c connecting the ends of the incision 111 and the bottom point 111b of the incision 111. As a result, by way of the increased depth of the incision 111, which serves as the distance D between the virtual line 111c connecting the ends of the incision 111 and the bottom point 111b of the incision 111, it becomes easy for the front end of the dull needle 115 which has reached the puncture hole 109 to enter the puncture hole 109.

Further, in the above embodiment, the inclined surface of the side part 10c is inclined such that the outer part is relatively low. However, such inclined surface may be inclined such that the outer part is relatively high.

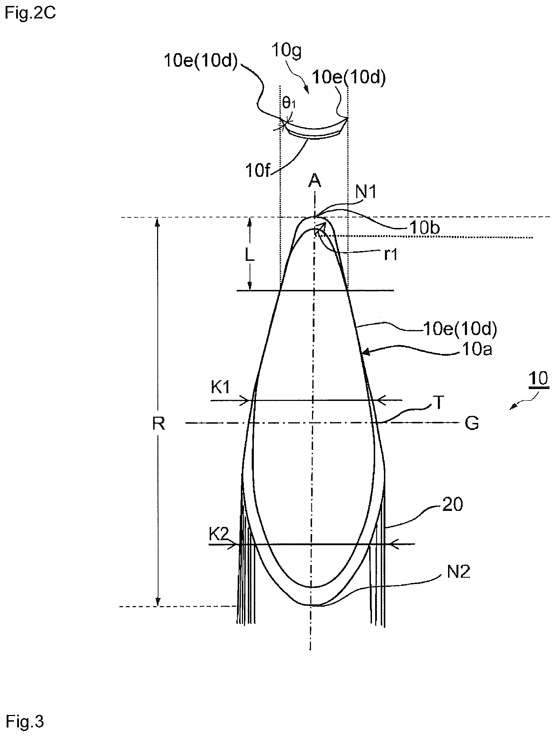

Moreover, the inclined side part 10c in the above embodiment may not be provided and, as shown in Fig. 2C, the sharp cutting lines 10e may be formed directly at the right and left side edges 10d contiguous with the arch-shaped front end part 10b of the puncture needle 10. In such case, the cutting lines 10e may be formed by sharpening, for example, an outer part of the inclined end face 10a.

A preferred embodiment of the present invention has been described above with reference to the attached drawings, but the present invention is not limited to such example. It is obvious that a person skilled in the art could conceive of various changes or modifications within the scope of the concepts stated in the claims, and such changes or modifications are naturally understood as falling within the technical scope of the present invention.

For example, in the above embodiment, during the first puncture, the curved-and-pointed end needle 120 is used to make the puncture hole 109 in the shunt vessel surface 104; during the second puncture operation, the puncture needle 10 is used to deepen the puncture hole 109; during the third puncture operation, the dull needle 115 is used, for the first time, to perform such puncture operation; and the subsequent puncture operations are continuously performed using the dull needle 115. However, it is not necessarily the case that the operation for deepening the puncture hole 109 with the use of the puncture needle 10 has to be performed as the second operation. The case may be such that, during the first puncture, the curved-and-pointed end needle 120 is used to make the puncture hole 109 in the shunt vessel surface 104; during the second and subsequent puncture operations, the dull needle 115 is used to continue such puncture operations; and then, after the puncture route 105 is established, the puncture needle 10 is used to deepen the puncture hole 109. In such case, even if the puncture needle 10, which is thicker than the curved-and-pointed end needle 120 used in the first puncture or the dull needle 115 used until just before, is used, because the puncture route 105 has been established, it will not be difficult for the front end of the puncture needle 10 to be inserted into the entrance 105a of the puncture route 105 on the skin surface.

Further, for example, in the above embodiment, the puncture needle 10 has been used to expand the puncture hole formed by the arc-shaped incision that is convex forward in the puncture direction. However, such puncture hole formed by the arc-shaped incision that is convex forward in the puncture direction is not the only thing that can be expanded using the puncture needle 10. No problem can be found in using the puncture needle 10 to expand a puncture hole formed by an arc-shaped incision that is convex backward in the puncture direction. Even if a puncture hole has an arch shape that is convex backward in the puncture direction, as long as the distance D between the virtual line 106a connecting the ends of the incision 106 and the bottom point 106b of the incision 106, as is shown in Fig. 12, it becomes easy for the front end of the dull needle 115 to enter the puncture hole 107.

It should be noted that a puncture method can also be proposed from another perspective, the puncture method comprising: a first puncture step of puncturing a shunt vessel through a skin surface so as to form a puncture route between the skin surface and a shunt vessel surface and to make an incision in the shunt vessel surface; and a second puncture step of expanding the incision in the shunt vessel surface which has been made in the first puncture step through the insertion into the shunt vessel via the puncture route from the skin surface and further via the puncture hole formed from the incision formed in the first puncture step.

Further, in such puncture method, the puncture in the first puncture step is performed using the pointed-and-curved end needle 112, and the puncture in the second puncture step is performed using the puncture needle 10. Moreover, such puncture method may comprise the third step of the dull needle 115 puncturing the shunt vessel through the expanded incision.

### Examples

### (Evaluation Test 1)

The intention of this test was to confirm that, in the case of making, in a shunt vessel surface, an arc-shaped incision that is convex forward in a puncture direction, the greater the angle formed by the puncture direction of the curved-and-pointed end needle 120 for forming an incision and a shunt vessel wall becomes, the smaller the opening area of a made puncture hole becomes; meanwhile, the smaller such angle formed by the puncture direction and the shunt vessel wall becomes, the greater the area of such puncture hole becomes. Namely, in evaluation test 1, a 17G curved-and-pointed end needle 120 was used to puncture a silicone rubber plate at angles of 20°, 30° and 40° with respect to a surface thereof while the inclined end face of such needle faced downward, thereby respectively making arc-shaped incisions convex forward in the puncture direction. Fig. 9 shows incisions P1, P2 and P3, on a silicone rubber plate, which are made as described above.

The increasing puncture angles of the curved-and-pointed end needle 120, i.e., 20°, 30° and 40° resulted in decreasing distances D between the virtual line 111c connecting the ends of the incision and the bottom point 111b of the incision, i.e., 0.951 mm, 0.809 mm and 0.727 mm.

### (Evaluation Test 2)

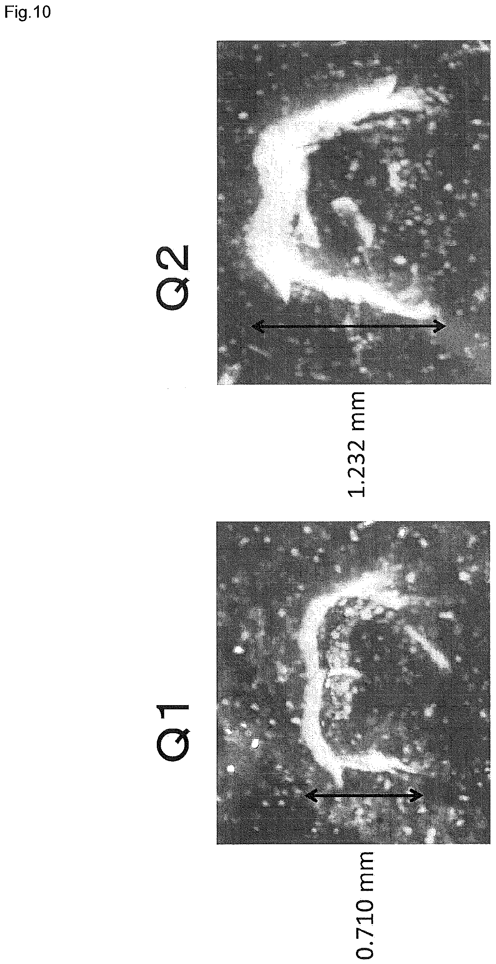

This test was aimed at confirming, in the case in which: the curved-and-pointed end needle 120 is used to puncture a shunt vessel surface at an angle of 30°, which is a general puncture angle, so as to make a convex incision; and the puncture needle 10 (edge angle of the cutting face: 58 degrees), which is thicker than the curved-and-pointed end needle is reinserted into the puncture hole formed by such incision, at 30° in the same manner as the above, whether or not the distance between a virtual line connecting both ends of the incision and the bottom point of the incision is extended through the second puncture with the puncture needle 10. In the evaluation test 2, in order to achieve such aim, firstly, the 17G (thickness) curved-and-pointed end needle 120 was used to puncture a silicone rubber plate at an angle of 30° with respect to a surface thereof while the inclined end face of such needle faced downward, thereby making an incision in the shunt vessel surface. Subsequently, a 15G puncture needle 10, which is thicker than the curved-and-pointed end needle 120, was inserted into the incision at an angle of 30°. Fig. 10 shows the photographs of a convex incision (Q1) made through the first puncture at a puncture angle of 30° with the use of the curved-and-pointed end needle 120 and an incision (Q2) formed after the insertion of the thicker puncture needle 10 into the above incision at an angle of 30°.

In the case in which a convex incision was made by using the curved-and-pointed end needle 120 to puncture a silicone rubber plate at an angle of 30° with respect to a surface thereof while the inclined end face of such needle faced downward (Q1), as to such incision which is convex forward in the puncture direction, the distance between the bottom point of the incision and the virtual line connecting both ends of the incision was 0.710 mm. Further, in the case in which the puncture needle 10, which is thicker than the curved-and-pointed end needle 120, was inserted into such incision at the same angle of 30° (Q2), the distance between the bottom point of the incision and the virtual line connecting both ends of the incision was 1.232 mm. That is, the above test results show that, as to an incision which is convex forward in the puncture direction and which has been made through a puncture at an angle of 30° with respect to the surface with the use of the curved-and-pointed end needle 120 while the inclined end face thereof is facing downward, the puncture needle 10, which is thicker than the curved-and-pointed end needle 120, is inserted into such incision at the same angle, thereby being capable of extending the distance between the bottom point of the incision and the virtual line connecting both ends of the incision.

### (Evaluation Test 3)

Evaluation test 3 was carried out so as to confirm whether or not it actually became easy to perform an insertion of a dull needle in the case in which, as to a puncture hole on a shunt vessel wall, wherein such hole was convex forward in a puncture direction and was made by puncturing a shunt vessel at an angle of 30° with respect to a surface thereof, the thicker puncture needle 10 was inserted, similarly at an angle of 30°, into such puncture hole so as to extend the incision.

Evaluation test 3 was carried out for 20 end-stage renal failure hemodialysis patients each having, in his or her forearm, a shunt inside an autologous vessel when performing the second session of blood purification treatment that was to be carried out three times a week. For each such patient, the 17G curved-and-pointed end needle 120 shown in Fig. 15 was used to penetrate the hypodermis at an angle of 30° with respect to a shunt vessel wall and then to puncture the shunt vessel through the skin while the inclined end face 120a of such curved-and-pointed end needle was facing downward, thereby forming a puncture route between the skin and the shunt vessel wall and also making a puncture hole on a shunt vessel surface on the extension of the puncture route. Such puncture was performed at two different locations. The shape of the resultant puncture holes is considered to be the convex forward arc shape shown in Fig. 14. On the day when such puncture holes were made, blood purification treatment was carried out by removing and returning blood through the curved-and-pointed end needle 120, which was inserted into the shunt vessel and placed therein in the above-described manner.

During the puncture operation in the next session of blood purification treatment performed two days later, regarding one of the above puncture holes made at two locations, as shown in Fig. 7, an experienced nurse inserted, into the puncture route 105 through the entrance 105a thereof on the skin 102, a needle having a tubular part thereof equal in radius to the curved-and-pointed end needle 120, i.e., the 17G dull needle 115, while the inclined end face located at the front end of such needle faced upward, and such nurse then moved the dull needle 115 forward while it maintained the posture along the puncture route 105 and then inserted the dull needle 115 into the shunt vessel 103 through the puncture hole 109.

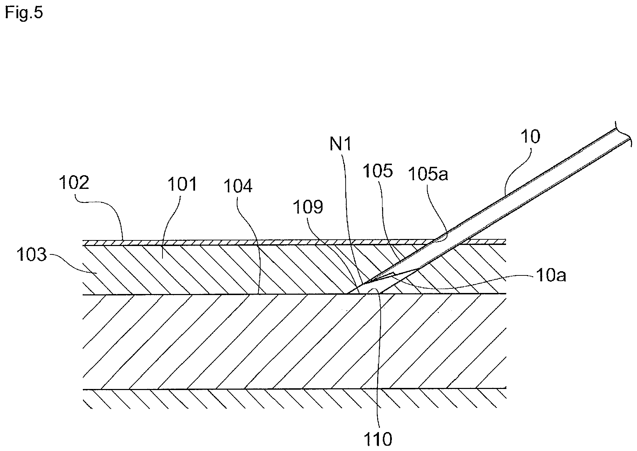

Further, regarding the other puncture hole, as shown in Fig. 5, the nurse inserted, into the puncture route 105 through the entrance 105a thereof on the skin 102, the 15G puncture needle 10 (edge angle of the cutting surface: 39 degrees), having a tubular part thereof thicker in diameter to the curved-and-pointed end needle 120, while the inclined end face 10a located at the front end of the puncture needle 10 was facing downward, and such nurse then moved the puncture needle 10 forward while maintaining the posture along the puncture route 105. As a result, when the front end part 10b of the puncture needle 10 reached the puncture hole 109 on the shunt vessel surface 104, the cutting lines 10e forming the side edges 10d of the side parts 10c of the puncture needle 10 dissected the ends of the incision 111 of the puncture 109 backward in the puncture direction X, whereby the puncture needle 10 was inserted into the puncture hole while maintaining its posture so as to extend the incision 111 backward in the puncture direction X. Thereafter, blood purification treatment was carried out by removing or returning blood through the puncture needle 10 placed inside the shunt vessel 103 in the above-described manner.

Subsequently, during the puncture operation in the next blood purification treatment session performed two days later, a nurse, who was different from the nurse who performed the previous puncture of the shunt vessel 103 with the use of the dull needle 115 and the puncture needle 10, used the 17G dull needle 115 with respect to the puncture hole that had not been expanded by the puncture needle 10, while such nurse used the 15G dull needle 115 with respect to the puncture hole which had already been expanded by the puncture needle 10, so as to puncture the shunt vessel through the respective puncture holes 109. Further, such nurse was requested to make a report concerning whether or not he or she had felt resistance when inserting the respective dull needles 115 into the shunt vessel 103 through the respective puncture holes 109. The results of evaluation test 3 are shown in Table 1 below.

### [Table 1]

**Table 1. Results of Evaluation Test 3**

| | "Resistance" | "Non-resistance" |
|---|---|---|
| Non-extension of a puncture hole by the puncture needle 10 | 8 (40%) | 12 (60%) |
| Extension of a puncture hole by the puncture needle 10 | 2 (10%) | 18 (90%) |

In the case in which the incision 111 was extended by the puncture needle 10 according to the present invention, the ratio of "non-resistance" was increased regarding the insertion of the dull needle 115.

### Description of Reference Numerals

- 1: Blood purification treatment device
- 11: Tube
- 14: Clamp
- 15: Cap
- 10: Puncture needle
- 10a: Inclined end face
- 10b: Front end part
- 10c: Side part
- 10d: Side edge
- 10e: Cutting line
- 10f: Outer surface
- 10g: Virtual cross-section
- 20: Tubular part
- N2: Back end
- N1: Front end
- T: End point of back end
- G: Virtual transverse line
- R: Distance
- 101: Hypodermis
- 102: Skin
- 103: Shunt vessel
- 104: Shunt vessel surface
- 105: Puncture route
- 105a: Entrance
- 106: Incision
- 106a: Virtual line
- 106b: Bottom point
- 107, 109: Puncture hole
- 108,110: Flap
- 108b: Optimum pressure-application part
- 111: Incision
- 111b: Bottom point of arc
- 111c: Chord of arc
- 115: Dull needle
- 115a: Inclined end face
- 115b: Front end part
- 115c: Side part
- 115d: Side edge
- 115f: Outer surface
- 115g: Virtual cross-section
- 115q: Front end
- A: Long axis
- L: Distance
- 120: Curved-and-pointed end needle
- 120a: Inclined end face
- 120b: Pointed end point
- Y: Centerline

## Claims

1. A puncture needle (10) for insertion into a puncture route (105) formed to cover from a skin surface to a shunt vessel surface (104) so as to expand an incision (111) forming a puncture hole (107, 109) that is made in the shunt vessel surface (104), the puncture needle (10) comprising:
an inclined end face (10a) that is inclined toward a front end of the needle (10) and has a front end part (10b), and
wherein right and left side edges (10d) of the inclined end face (10a) which are contiguous with the front end part (10b) are provided with cutting lines (10e) each having an edge angle (θ1) from 5 degrees to 85 degrees,
**characterized in that** the front end part (10b) of the inclined end face (10a) constitutes an edge having no sharpness and is formed into an arc shape.

2. The puncture needle (10) according to claim 1, wherein right and left side parts (10c) of the inclined end face (10a) which are contiguous with the front end part (10b) are respectively inclined, and side edges (10d) of the side parts (10c) are provided with the cutting lines (10e).

3. The puncture needle (10) according to claim 2, wherein the side parts (10c) are inclined so as to become lower in an outward direction.

4. The puncture needle (10) according to claim 1, wherein the right and left side edges are provided with sharp cutting lines (10e) and cutting lines (10e) are formed by sharpening an outer part of the inclined end face (10a).

5. The puncture needle (10) according to any of claims 1 to 4, wherein the edge angle of the cutting lines (10e) is from 15 degrees to 65 degrees.

6. The puncture needle (10) according to claim 5, wherein the front end part (10b) of the inclined end face (10a) has an arc-direction length of 0.05 mm or more and less than 1.2 mm.

7. The puncture needle (10) according to claim 5 or 6, wherein the arc-shaped front end part (10b) of the puncture needle (10) has a radius of curvature (r2) of 200 µm or more and less than 4.4 mm.

8. The puncture needle (10) according to any of claims 1 to 7, wherein start points of the cutting lines (10e) are located at boundaries between the front end part (10b) of the inclined end face (10a) and the side parts (10c) thereof, and end points of the cutting lines (10e) are located at positions where a virtual transverse line (G) passing through a point that falls, with respect to a front end (N1) of the inclined end face (10a), within the range of one-tenth to seven-tenths of a distance (A) that covers from the front end (N1) to a back end (N2) of the inclined end face (10a), intersects with the side edges (10d) of the inclined end face (10a).

9. The puncture needle (10) according to any of claims 1 to 8, wherein the puncture needle (10) is inserted into the puncture hole (107, 109) after the puncture hole (107, 109) is made.

10. The puncture needle (10) according to any of claims 1 to 9, wherein the puncture needle (10) has a diameter greater than that of a puncture needle used for making the puncture hole.

11. The puncture needle (10) according to claim 2 or 3, wherein an inclined surface of each of the side parts (10c) which is inclined in an outward direction is a flat surface.

12. The puncture needle (10) according to claim 2 or 3, wherein the needle outer-diameter width of a part of an inclined surface of each of the side parts (10c) which is inclined in an outward direction is smaller than the needle outer-diameter width of a part behind the part of the inclined surface.

13. The puncture needle (10) according to claim 2 or 3, wherein an inclination angle of an inclined surface of each of the side parts (10c) is more than 0 degree and equal to or less than 30 degrees.

14. The puncture needle (10) according to any of claims 1 to 13, wherein, regarding the edge having no sharpness of the front end part (10b), the front end part (10b) has a radius of curvature (r2) of 0.01 mm to 0.05 mm when seen from a side surface of the needle (10).

## Patentansprüche

1. Punktionsnadel (10) zum Einfügen in eine Punktionsbahn (105), die gebildet wurde, um den Weg von einer Hautoberfläche zu einer Shuntgefäßoberfläche (104) abzudecken, zum Aufweiten eines Einschnitts (111), der ein Punktionsloch (107, 109) bildet und in die Shuntgefäßoberfläche (104) gemacht wurde, wobei die Punktionsnadel (10) umfasst:
eine abgeschrägte Endfläche (10a), die gegenüber einem Vorderende der Nadel (10) abgeschrägt ist und einen Vorderendeteil (10b) aufweist, wobei die rechtsseitige und die linksseitige Kante (10d) der abgeschrägten Endfläche (10a), die mit dem Vorderendeteil (10b) zusammenhängen, mit Schneidelinien (10e) versehen sind, die jeweils einen Kantenwinkel (θ1) von 5 Grad bis 85 Grad aufweisen,
**dadurch gekennzeichnet, dass** der Vorderendeteil (10b) der abgeschrägten Endfläche (10a) eine Kante bildet, die keine Schärfe aufweist und in einer Bogenform ausgebildet ist.

2. Punktionsnadel (10) gemäß Anspruch 1, wobei ein rechtsseitiger und ein linksseitiger Teil (10c) der abgeschrägten Endfläche (10a), die mit dem Vorderendeteil (10b) zusammenhängen, jeweils abgeschrägt sind und Seitenkanten (10d) der Seitenteile (10c) mit den Schneidelinien (10e) versehen sind.

3. Punktionsnadel (10) gemäß Anspruch 2, wobei die Seitenteile (10c) so abgeschrägt sind, dass sie in einer nach außen gerichteten Richtung niedriger werden.

4. Punktionsnadel (10) gemäß Anspruch 1, wobei die rechtsseitige und die linksseitige Kante mit scharfen Schneidelinien (10e) versehen sind und die Schneidelinien (10e) dadurch gebildet werden, dass man einen äußeren Teil der abgeschrägten Endfläche (10a) schärft.

5. Punktionsnadel (10) gemäß einem der Ansprüche 1 bis 4, wobei der Kantenwinkel der Schneidelinien (10e) 15 Grad bis 65 Grad beträgt.

6. Punktionsnadel (10) gemäß Anspruch 5, wobei der Vorderendeteil (10b) der abgeschrägten Endfläche (10a) eine Länge in Bogenrichtung von 0,05 mm oder mehr und weniger als 1,2 mm aufweist.

7. Punktionsnadel (10) gemäß Anspruch 5 oder 6, wobei der bogenförmige Vorderendeteil (10b) der Punktionsnadel (10) einen Krümmungsradius (r2) von 200 µm oder mehr und weniger als 4,4 mm aufweist.

8. Punktionsnadel (10) gemäß einem der Ansprüche 1 bis 7, wobei sich Startpunkte der Schneidelinien (10e) an Grenzen zwischen dem Vorderendeteil (10b) der abgeschrägten Endfläche (10a) und deren Seitenteilen (10c) befinden und sich Endpunkte der Schneidelinien (10e) auf Positionen befinden, wo sich eine virtuelle Querlinie (G), die durch einen Punkt, der in Bezug auf ein Vorderende (N1) der abgeschrägten Endfläche (10a) in den Bereich von einem Zehntel bis sieben Zehntel des Abstands (A) fällt, der vom Vorderende (N1) bis zu einem hinteren Ende (N2) der abgeschrägten Endfläche (10a) verläuft, mit den Seitenkanten (10d) der abgeschrägten Endfläche (10a) schneidet.

9. Punktionsnadel (10) gemäß einem der Ansprüche 1 bis 8, wobei die Punktionsnadel (10) in das Punktionsloch (107, 109) eingefügt wird, nachdem das Punktionsloch (107, 109) gebildet wurde.

10. Punktionsnadel (10) gemäß einem der Ansprüche 1 bis 9, wobei die Punktionsnadel (10) einen größeren Durchmesser aufweist als die zur Bildung des Punktionslochs verwendete Punktionsnadel.

11. Punktionsnadel (10) gemäß Anspruch 2 oder 3, wobei die abgeschrägte Fläche jedes der Seitenteile (10c), die in einer nach außen gerichteten Richtung abgeschrägt ist, eine flache Fläche ist.

12. Punktionsnadel (10) gemäß Anspruch 2 oder 3, wobei die Nadelaußendurchmesserbreite eines Teils einer abgeschrägten Fläche jedes der Seitenteile (10c), der in einer nach außen gerichteten Richtung abgeschrägt ist, kleiner ist als die Nadelaußendurchmesserbreite eines Teils hinter dem Teil der abgeschrägten Fläche.

13. Punktionsnadel (10) gemäß Anspruch 2 oder 3, wobei der Abschrägungswinkel einer abgeschrägten Fläche jedes der Seitenteile (10c) größer als 0 Grad und kleiner oder gleich 30 Grad ist.

14. Punktionsnadel (10) gemäß einem der Ansprüche 1 bis 13, wobei bezüglich der Kante des Vorderendeteils (10b), die keine Schärfe aufweist, der Vorderendeteil (10b) einen Krümmungsradius (r2) von 0,01 mm bis 0,05 mm aufweist, wenn er von einer Seitenfläche der Nadel (10) aus betrachtet wird.

## Revendications

1. Aiguille de ponction (10) pour insertion dans un trajet de ponction (105) formé à partir d'une surface de la peau jusqu'à la surface (104) d'un vaisseau sanguin dérivé de façon à élargir une incision (111) formant un orifice de ponction (107, 109) réalisé dans la surface (104) du vaisseau sanguin dérivé, l'aiguille de ponction (10) comprenant :
une face d'extrémité inclinée (10a), laquelle est inclinée vers une extrémité avant de l'aiguille (10) et présente une partie d'extrémité avant (10b),
les bords latéraux droite et gauche (10d) de la face d'extrémité inclinée (10a), qui sont contigus à la partie d'extrémité avant (10b), sont pourvus de lignes de coupe (10e) ayant chacune un angle de bord θ1 de 5 à 85 degrés,
**caractérisée en ce que** la partie d'extrémité avant (10b) de la face d'extrémité inclinée (10a) forme un bord non tranchant et a la forme d'un arc.

2. Aiguille de ponction (10) selon la revendication 1, dans laquelle les parties latérales droite et gauche (10c) de la face d'extrémité inclinée (10a), qui sont contiguës à la partie d'extrémité avant (10b) sont respectivement inclinées, et les bords latéraux (10d) des parties latérales (10c) sont pourvus des lignes de coupe (10e).

3. Aiguille de ponction (10) selon la revendication 2, dans laquelle les parties latérales (10c) sont inclinées de façon à s'abaisser vers l'extérieur.

4. Aiguille de ponction (10) selon la revendication 1, dans laquelle les bords latéraux droite et gauche sont pourvus de lignes de coupe tranchantes (10e) et les lignes de coupe (10e) sont formées en affûtant une partie extérieure de la face d'extrémité inclinée (10a).

5. Aiguille de ponction (10) selon l'une des revendications 1 à 4, dans laquelle l'angle de bord des lignes de coupe (10e) est compris entre 15 et 65 degrés.

6. Aiguille de ponction (10) selon la revendication 5, dans laquelle la partie d'extrémité avant (10b) de la face d'extrémité inclinée (10a) a une longueur en direction de l'arc égale à 0,05 mm ou plus et inférieure à 1,2 mm.

7. Aiguille de ponction (10) selon la revendication 5 ou 6, dans laquelle la partie d'extrémité avant en forme d'arc (10b) de l'aiguille de ponction (10) a un rayon de courbure (r2) égal à 200 µm ou plus et inférieur à 4,4 mm.

8. Aiguille de ponction (10) selon l'une des revendications 1 à 7, dans laquelle les points de départ des lignes de coupe (10e) sont situés aux limites entre la partie d'extrémité avant (10b) de la face d'extrémité inclinée (10a) et les parties latérales (10c) de celle-ci, et les points terminaux des lignes de coupe (10e) sont situés à des positions où une ligne transversale virtuelle (G), traversant un point qui se situe, par rapport à une extrémité avant (N1) de la face d'extrémité inclinée (10a), dans la plage de un dixième à sept dixièmes d'une distance (A) entre l'extrémité avant (N1) et une extrémité arrière (N2) de la face d'extrémité inclinée (10a), coupe les bords latéraux (10d) de la face d'extrémité inclinée (10a).

9. Aiguille de ponction (10) selon l'une des revendications 1 à 8, dans laquelle l'aiguille de ponction (10) est insérée dans l'orifice de ponction (107, 109) après réalisation de l'orifice de ponction (107, 109).

10. Aiguille de ponction (10) selon l'une des revendications 1 à 9, dans laquelle l'aiguille de ponction (10) a un diamètre supérieur à celui d'une aiguille de ponction utilisée pour réaliser l'orifice de ponction.

11. Aiguille de ponction (10) selon la revendication 2 ou 3, dans laquelle une surface inclinée de chacune des parties latérales (10c) qui est inclinée vers l'extérieur est une surface plane.

12. Aiguille de ponction (10) selon la revendication 2 ou 3, dans laquelle la largeur de diamètre extérieur d'aiguille d'une partie d'une surface inclinée de chacune des parties latérales (10c) qui est inclinée vers l'extérieur est plus petite que la largeur de diamètre extérieur d'aiguille d'une partie située derrière la partie de la surface inclinée.

13. Aiguille de ponction (10) selon la revendication 2 ou 3, dans laquelle un angle d'inclinaison d'une surface inclinée de chacune des parties latérales (10c) est supérieur à 0 degré et égal ou inférieur à 30 degrés.

14. Aiguille de ponction (10) selon l'une des revendications 1 à 13, dans laquelle, en ce qui concerne le bord non tranchant de la partie d'extrémité avant (10b), la partie d'extrémité avant (10b) a un rayon de courbure (r2) de 0,01 mm à 0,05 mm, vue à partir d'une surface latérale de l'aiguille (10).
